# NOUVEAU FASCICULE DE BREVET EUROPEEN

(11) **EP 1 473 016 B2**
(45) Date de publication et mention de la décision concernant l'opposition: **10.08.2016**
(45) Mention de la délivrance du brevet: 02.11.2006
(21) Numéro de dépôt: 04291083.6
(22) Date de dépôt: 27.04.2004
(51) Int. Cl.: A61K 8/06, A61K 8/81, A61K 8/898, A61Q 1/00

(54) **Compositions cosmétiques de type émulsion solide eau-dans-huile**
Kosmetische Zusammensetzungen des Typs feste Wasser-in-Öl Emulsion
Cosmetic compositions of the water-in-oil solid emulsion type

(30) Priorité: 30.04.2003 FR 0305326
(43) Date de publication de la demande: 03.11.2004
(73) Titulaire: L'Oréal, 75008 Paris (FR)
(72) Inventeur: Auguste, Frédéric, 94550 Chevilly-Larue (FR); Portois, Emmanuelle, 92320 Chatillon (FR)
(74) Mandataire: Nony

(56) Documents cités:
- EP-A1- 0 374 332
- US-A- 5 876 704
- US-A1- 2002 106 385
- Brochure "Dow Corning Silicone Emulsifiers" / DC 9011 et DC5200
- Fiche technique DC2501
- Fiche technique Wacker-Belsil DMC 6038
- Fiche technique DC AMS-C30

## Description

La présente invention concerne le domaine d'émulsions solides eau-dans-huile de soin et/ou de traitement et/ou de maquillage de la peau, y compris du cuir chevelu, et/ou des lèvres des êtres humains, se présentant notamment sous forme d'un produit coulé de maquillage et en particulier d'un stick de maquillage comme les rouges à lèvres ou les fonds de teint. Il peut notamment s'agir de compositions de maquillage et/ou de soin de la peau et/ou des lèvres, de compositions solaires et de compositions d'hygiène comme les déodorants.

Dans le domaine cosmétique, les émulsions eau-dans-huile sont couramment utilisées car elles permettent notamment de véhiculer des actifs dans la phase aqueuse et d'apporter une sensation de fraîcheur lors de l'application et après l'application. Les émulsions eau-dans-huile conventionnelles contiennent un ou plusieurs tensioactifs et une phase huileuse. Elles peuvent aussi comprendre une phase cireuse. La phase cireuse sert notamment à structurer l'émulsion eau-dans-huile notamment pour en obtenir un stick. Pour ce faire, les particules de cires créent entre elles un réseau établi par connexion des particules de cires les unes aux autres et c'est ce réseau qui assure la cohésion du produit.

D'une manière générale, les émulsions eau-dans-huile solides conventionnelles ne peuvent pas contenir une proportion élevée de phase dispersée aqueuse, c'est-à-dire plus de 50 % en poids sous peine d'affecter significativement les propriétés mécaniques attendues.

Ainsi la demande de brevet EP 1 064 908 décrit des compositions cosmétiques solides de type émulsion inverse contenant moins de 30 % en poids d'eau stabilisée par un agent émulsifiant de la famille des carboxy-alkyl-polyglycérols, et une phase grasse dont le point de liquéfaction est supérieur à 60°C. La demande de brevet JP-A-03 261 707 décrit des émulsions cosmétiques solides contenant des huiles de silicone, des cires de point de fusion égal à 80°C, de l'eau et au moins un agent émulsifiant de type diméthicone copolyol. La demande de brevet WO 99/ 47111 décrit des compositions solides cosmétiques du type émulsion eau-dans-huile comprenant moins de 40 % en poids d'une phase aqueuse et émulsionnée, à l'aide d'un tensio-actif siliconé de type alkyldiméthicone copolyol, dans une phase grasse contenant une cire de polyéthylène et une cire de jojoba hydrogénée dont les points de fusion sont de l'ordre de 70°C.

Plus récemment, la demande de brevet EP 1 159 954 propose d'utiliser dans des émulsions inverses solides, la cire de jojoba hydrogénée en dispersion dans une phase aqueuse, présente à raison de 5 à 50 % en poids, ladite émulsion étant stabilisée à l'aide d'au moins un tensio-actif siliconé de type polyoxyalkylène.

De manière inattendue, les inventeurs ont constaté qu'il était possible d'utiliser efficacement, à titre d'agent texturant dans une émulsion eau-dans-huile solide, une cire spécifique caractérisée par sa température de fusion et par son aptitude à se présenter à température ambiante sous la forme de cristallites de forme spécifique. Avantageusement, une telle cire permet d'obtenir des compositions cosmétiques sous forme de sticks non cassants suffisamment durs jusqu'à des taux très élevés de phase aqueuse. Les compositions correspondantes permettent également un dépôt sur les matières kératiniques suffisant.

Plus précisément, la présente invention concerne, selon un premier de ses aspects, une composition cosmétique sous la forme d'une émulsion solide eau-dans-huuile comprenant une phase aqueuse dispersée dans une phase grasse caractérisée en ce qu'elle comprend plus de 60% en poids de phase aqueuse et en ce que ladite phase grasse comprend au moins une cire dont la température de fusion est comprise entre 25 °C et 42 °C, et qui se trouve à l'état solide sous la forme de cristallites possédant un facteur de forme au moins égal à 2 et une longueur moyenne comprise entre 20µm et 50µm.

La présente invention concerne également, selon un autre de ses aspects, l'utilisation d'au moins une cire de température de fusion comprise entre 25°C et 42 °C, se présentant à l'état solide sous la forme de cristallites possédant un facteur de forme au moins égal à 2 et une longueur moyenne comprise entre 20µm et 50µm à titre d'agent texturant pour la préparation d'une composition cosmétique se présentant sous la forme d'une émulsion solide eau-dans-huile comprenant plus de 60% en poids de phase aqueuse.

La présente invention décrit en outre, selon un autre de ses aspects, l'utilisation d'au moins une cire se présentant à l'état solide sous la forme de cristallites possédant un facteur de forme au moins égal à 2, à titre d'agent texturant pour ta préparation d'une composition cosmétique sous la forme d'une émulsion solide eau-dans-huile contenant plus de 50 % en poids d'une phase aqueuse.

La présente invention vise également un procédé cosmétique de soin et/ou de maquillage de la peau et/ou des lèvres comprenant l'application sur la peau et/ou les lèvres d'une composition conforme à l'invention.

Selon l'invention, les compositions considérées sont des émulsions eau-dans-huiles, c'est-à-dire obtenues par émulsification, à l'aide d'un ou plusieurs agents tensioactifs, d'une phase aqueuse dans une phase huileuse.

Au sens de l'invention, un agent texturant désigne un composé ou mélange de composés permettant d'obtenir une émulsion solide.

Par « solide », on entend que la mesure de la force maximale mesurée en texturométrie lors de l'enfoncement d'une sonde dans l'échantillon de formule doit être au moins égale à 0,25 Newton, en particulier au moins égal à 0,30 Newton, notamment au moins égale 0,35 Newton, appréciée dans des conditions de mesure précises comme suit.

Les formules sont coulées à chaud dans des pots de 4 cm de diamètre et 3 cm de fond. Le refroidissement est fait à température ambiante. La dureté des formules réalisées est mesurée après 24 heures d'attente. Les pots contenant les échantillons sont caractérisés en texturométrie à l'aide d'un texturomètre tel que celui commercialisé par la société Rhéo TA-XT2, selon le protocole suivant : une sonde de type bille en inox de diamètre 5 mm est amenée au contact de l'échantillon à une vitesse de 1 mm/s. Le système de mesure détecte l'interface avec l'échantillon avec un seuil de détection égal à 0,005 newtons. La sonde s'enfonce de 0,3 mm dans l'échantillon, à une vitesse de 0,1 mm/s. L'appareil de mesure enregistre l'évolution de la force mesurée en compression au cours du temps, pendant la phase de pénétration. La dureté de l'échantillon correspond à la moyenne des valeurs maximales de la force détectée pendant la pénétration, sur au moins 3 mesures.

Comme précisé précédemment, les inventeurs ont constaté que l'incorporation dans des émulsions eau-dans-huile, d'au moins une cire conforme à l'invention permettait de conférer simultanément à la formulation cosmétique correspondante une consistance avantageuse en terme de conditionnement et des propriétés au dépôt satisfaisantes, pour son application sur la surface à traiter et/ou maquiller, cette dernière propriété se traduisant notamment par une bonne aptitude à l'étalement.

Les propriétés de dépôt d'une composition selon l'invention sont appréciées visuellement par dépôt de la composition généralement sur une surface corporelle. Son étalement doit pouvoir être effectué facilement c'est à dire avec des propriétés de glissant satisfaisantes et permettre d'accéder rapidement à une bonne homogénéité d'épaisseur de dépôt sur l'ensemble de cette surface.

### Cire ou phase cireuse

Par "cire" au sens de la présente invention, on entend un composé lipophile à changement d'état solide/liquide réversible, ayant une température de fusion supérieure ou égale à 25 °C pouvant aller jusqu'à 200 °C, et présentant à l'état solide une organisation cristalline anisotrope. En fondant la cire, il est possible de la rendre miscible à des huiles et de former un mélange homogène microscopiquement, mais en abaissant la température du mélange, on obtient une recristallisation de la cire dans les huiles.

Avantageusement, la cire ou la phase cireuse considérée selon l'invention possède une température de fusion comprise entre 25 °C et 42 °C, notamment entre 25 °C et 40 °C et plus particulièrement entre 25 °C et 35 °C.

Au sens de l'invention, la température de fusion correspond à la température du pic le plus endothermique observé en analyse thermique (DSC) telle que décrite dans la norme ISO 11357-3; 1999.

Le point de fusion de la cire peut être mesuré à l'aide d'un calorimètre à balayage différentiel (DSC), par exemple le calorimètre vendu sous la dénomination MDSC 2920 par la société TA Instruments.

Le protocole de mesure est le suivant:

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de ta température. Le point de fusion du composé est la valeur de la température correspondant au sommet du pic de la courbe représentant la variation de la différence de puissance absorbée en fonction de la température.

Comme précisé précédemment, la cire se présente à l'état solide sous forme de cristallites de facteur de forme au moins égal à 2 que l'on peut encore qualifier de cristallites en aiguilles.

D'une manière générale, les cristallites en aiguilles sont des cristallites se présentant sous la forme d'objets dont une dimension est supérieure aux deux autres. Elles sont caractérisées par leur facteur de forme, c'est-à-dire le rapport de leur plus grande longueur sur la plus grande des deux autres dimensions (largeur, épaisseur). Dans le cadre de la présente invention, ce facteur de forme est supérieur ou égal à 2, en particulier supérieur ou égal à 3, plus particulièrement supérieur ou égal à 4 et notamment supérieur ou égal à 5.

Ces cristallites en aiguilles et notamment leurs dimensions peuvent être caractérisées visuellement selon la méthode suivante.

La cire est déposée sur une lame de microscope, laquelle est posée sur une platine chauffante. La lame et la cire sont chauffées à une température généralement au moins supérieure de 5 °C à celle du point de fusion,de la cire ou du mélange de cire considéré(e). A la fin de la fonte, le liquide ainsi obtenu et la lame de microscope sont laissés refroidir pour se solidifier. L'observation des cristallites est réalisée à l'aide d'un microscope optique de type Leica DMLB100, avec un objectif sélectionné en fonction de la taille des objets à visualiser, et en lumière polarisée. Les dimensions des cristallites sont mesurées à l'aide d'un logiciel d'analyse d'Images tel que ceux commercialisés par la société Microvision.

Par « longueur moyenne », on désigne la dimension donnée par la distribution granulométrique statistique à la moitié de la population, dite D50.

La longueur moyenne des cristallites est plus particulièrement déterminante pour les émulsions eau-dans-huile solides possédant une teneur élevée en phase aqueuse notamment supérieure à 50 % en poids par rapport au poids total de ladite composition.

En effet, lorsqu'il y a une phase dispersée en quantité importante c'est-à-dire supérieure à 50 % et pouvant atteindre jusqu'à 90 % en poids, les gouttelettes de la phase dispersée sont suffisamment proches les unes des autres pour empêcher une structuration convenable par le réseau des cires, si ces cires ne sont pas spécifiques en termes de taille et de facteur de forme. Grâce à leur forme allongée et leur taille généralement comparable à celle des gouttelettes aqueuses dispersées, les cristallites de cire selon l'invention peuvent avantageusement s'insérer entre les gouttelettes aqueuses et former ainsi le réseau de cire nécessaire à l'obtention des propriétés mécaniques requises au niveau de l'émulsion.

L'invention s'étend également aux compositions dans lesquelles ces cristallites sont associées à des cristallites de cire ne répondant pas aux critères de facteur de forme et/ou de température de fusion et/ou de taille définis précédemment.

En particulier, il peut s'agir de cristallites qui possèdent un facteur de forme et/ou une longueur conforme à l'invention mais une température de fusion ne correspondant pas à la plage de fusion préférée ou requise selon l'invention. Il peut encore s'agir de cristallites possédant un facteur de forme différent de celui requis selon l'invention.

Au sens de l'invention, une cire ne pouvant se présenter sous forme de cristallites possédant un facteur de forme au moins égal à 2 et une longueur moyenne comprise entre 20µm et 50µm et/ou ne possédant pas une température de fusion variant de 25° à 42 °C sera désignée sous le terme de « cire non conforme à l'invention » ou encore de « cire conventionnelle ».

D'une manière générale, au moins une cire conforme à l'invention est présente en quantité suffisante dans ladite composition pour conférer à celle-ci la texture et les propriétés mécaniques attendues. C'est ce que l'on entend définir au sens de l'invention par le terme « quantité efficace ».

Ainsi, les compositions cosmétiques contiennent au moins une cire à l'état de cristallites conforme à l'invention en quantité suffisante pour que la force maximale mesurée en texturométrie lors de l'enfoncement d'une sonde dans un échantillon de celle-ci selon les conditions de mesure précises définies précédemment doit être au moins égale à 0,25 Newton, en particulier au moins égal à 0,30 Newton.

Par exemple, les compositions selon l'invention peuvent contenir de 1 à 20 % notamment de 2 à 20 %, en particulier de 4 à 10 % en poids de cire conforme à l'invention.

Toutefois, pour les raisons explicitées précédemment, la quantité efficace de cire conforme à l'invention, à incorporer pour obtenir la dureté requise et les propriétés d'étalement souhaitées est susceptible de varier significativement selon la quantité de phase continue (cire et huile) et de phase dispersée (phase aqueuse). De plus, cette quantité est également susceptible de varier selon que la composition comprend ou non en outre une ou plusieurs autre(s) cire (s) dite(s) conventionnelle(s), et en fonction des paramètres physiques de ces cires tels que par exemple dureté et enfin de leurs quantités respectives.

Selon une variante, les compositions selon l'invention peuvent comprendre une phase grasse pouvant comprendre de 10 à 40 % en poids, notamment de 10 à 30 % en poids, en particulier de 5 à 25 % en poids d'une phase cireuse contenant de 50 à 100 % en poids, en particulier de 70 à 100 % en poids d'au moins une cire conforme à l'invention, et éventuellement de 0,5 à 50 % en poids, en particulier de 0,5 à 30 % en poids d'au moins une cire non conforme à l'invention.

Selon un mode de réalisation particulier de l'invention, la phase cireuse de la composition selon l'invention est constituée par une ou plusieurs cire(s) conforme(s) à l'invention.

Les cires convenant à l'invention peuvent être d'origine naturelle, notamment végétale, minérale ou animale et/ou synthétiques. Les cires peuvent être à la fois d'origine animale et synthétiques.

Il peut notamment s'agir de cires hydrocarbonées ou siliconées.

Les cires hydrocarbonées ont avantageusement une densité à 25°C inférieure à 0,9, de préférence Inférieure à 0,8 g/cm³, de préférence comprise entre 0,75 et 0,80 g/cm³. Elles ont également avantageusement une masse moléculaire inférieure à 500 g/mol, de préférence inférieure ou égale à 400 g/mol, de préférence encore comprise entre 200 et 400 g/mol, de préférence encore comprise entre 250 et 350 g/mol.

A titre illustratif et non limitatif de cires hydrocarbonées, on peut plus particulièrement citer les cires de Fischer-Tropsch, également appelées cires de polyméthylène ou cire de paraffine synthétique. Elles répondent à la formule CnH2n+2.

Selon un mode de mise en oeuvre particulier de l'invention, la cire selon invention est au moins une cire de polyméthylène et en particulier la cire Cirebelle 505^{®}, fabriquée par la société SASOL, dont le point de fusion est égal à 40 °C.

En ce qui concerne les cires siliconées, il peut notamment s'agir d'une cire de type silicone polyoxyalkylénée c'est-à-dire une silicone comportant au moins un groupement oxyalkyléné de type (-CₓH₂ₓO)ₐ dans lequel x peut varier de 2 à 6 et a est supérieur à ou égal à 2.

Les silicones oxyalkylénées susceptibles de convenir à l'invention peuvent être choisies parmi les composés de formules générales (I), (II), (III) ou (IV): 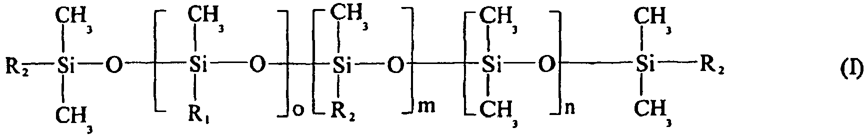 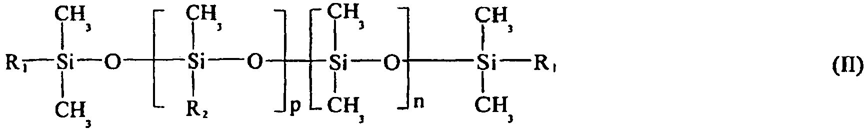 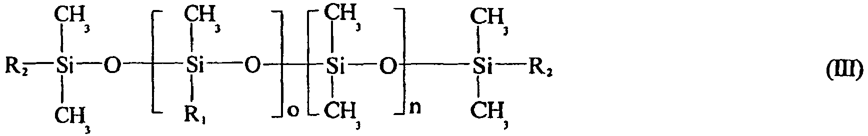 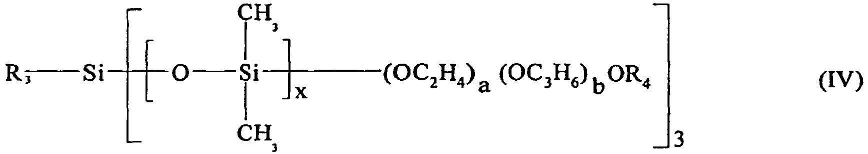 formules (I), (II), (III) et (IV) dans lesquelles :
- R₁, identique ou différent, représente un radical alkyle, linéaire ou ramifié, en C₁-C₃₀ ou phényle,
- R₂, identique ou différent, représente un radical C_{c}H_{2c}-O-(C₂H₄O)ₐ(C₃H₆O)_{b}-R₅ ou un radical -C_{c}H_{2c}-O-(C₄H₈O)ₐ-R₅,
- R₃ et R₄, identiques ou différents, désignent un radical alkyle, linéaire ou ramifié, en C₁ à C₁₂ et de préférence le radical méthyle,
- R₅, identique ou différent, est choisi parmi un atome d'hydrogène, un radical alkyle, linéaire ou ramifié, de 1 à 12 atomes de carbone, un radical alcoxy, linéaire ou ramifié, de 1 à 6 atomes de carbone, un radical acyle, linéaire ou ramifié, de 2 à 30 atomes de carbone, un radical hydroxyle, aminoalcoxy en C₁-C₆ éventuellement substitué sur l'amine, aminoacyle en C₂-C₆ éventuellement substitué sur l'amine, aminoalkyle éventuellement substitué sur l'amine et sur la chaîne alkyle, carboxyacyle en C₂-C₃₀, un groupement éventuellement substitué par un ou deux radicaux aminoalkyle substitués, - NHCO(CH₂)_{d}OH, un groupement phosphate,
- d varie de 1 à 10,
- m varie de 0 à 20,
- n varie de 0 à 500,
- o varie de 0 à 20,
- p varie de 1 à 50,
- a varie de 0 à 50,
- b varie de 0 à 50,
- a + b est supérieur ou égal à 2,
- c varie de 0 à 4,
- x varie de 1 à 100.

De telles silicones sont par exemple décrites dans les brevets US-A-5070171, US-A-5149765, US-A-5093452 et US-A-5091493.

Conviennent tout particulièrement, les silicones de formule (III) dans laquelle R₂, identique ou différent, représente un radical C_{c}H_{2c}O-(C₂H₄O)ₐ(C₃H₆O)_{b}-R₅, avec R₅, a, b et c étant définis comme précédemment. Dans ce mode de mise en oeuvre, b et c sont de préférence égaux à 0 et a est compris entre 1 et 50, de préférence entre 5 et 30, de préférence encore entre 10 et 20.

Bien entendu, les cires telles que définies ci-dessus doivent en outre manifester une aptitude à cristalliser à l'état de cristallites possédant un facteur de forme au moins égal à 2 et posséder une température de fusion variant de 25 °C à 42 °C.

A titre illustratif et non limitatif de ce type de cire, on peut plus particulièrement citer la cire Belsil DMC 6038^{®} commercialisée par la société WACKER-BELSIL.

### Phase aqueuse

La composition selon l'invention comprend à titre de phase dispersée au moins un milieu aqueux, constituant une phase aqueuse.

Cette phase aqueuse peut être constituée essentiellement d'eau.

Elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, et les aldéhydes en C₂-C₄.

Elfe peut également comprendre une dispersion de particules hydrophobes, comme par exemple des polymères en dispersion. L'homme de l'art veillera toutefois à ce que l'introduction d'épaississants polymères n'inverse pas le sens de l'émulsion.

Cette phase aqueuse peut, le cas échéant, être épaissie, gélifiée ou structurée en y incorporant en outre un gélifiant aqueux traditionnel notamment d'origine minérale comme l'argile par exemple et/ou organique comme un polymère gélifiant aqueux.

Comme précisé précédemment, cette phase aqueuse peut être présente en quantités très variables dans la composition selon l'invention. Elle représente plus de 60 % en poids, en particulier plus de 70 % en poids, notamment plus de 75 % en poids et plus particulièrement plus de 80 %, voire même plus de 85% en poids du poids total de la composition.

### Phase grasse

La composition selon l'invention peut comprendre, outre une phase cireuse, au moins une phase grasse liquide à température ambiante (25°C) et à pression atmosphérique. La phase grasse peut si nécessaire contenir en outre un ou plusieurs agents gélifiants et structurants d'huiles de nature organique et/ou des solvants organiques lipophiles.

La phase grasse liquide peut être présente à raison de 0,5 à 80 % en poids, en particulier de 1 à 75 % en poids, plus particulièrement de 2 à 65 % poids, notamment de 3 à 60 % en poids, voire de 5 à 50 % en poids par rapport au poids total de la composition salon l'invention.

La phase grasse de la composition selon l'invention peut notamment comprendre, à titre de phase grasse liquide, au moins un corps gras liquide de type huile volatile ou non, siliconée ou non, ou un de leurs mélanges.

Par « huile volatile », on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeurnon nulle, à température ambiante et pression atmosphérique, supérieure à 0,01 mm Hg et Inférieure à 300 mm de Hg (1,33 Pa à 40.000 Pa) et de préférence supérieure à 0,3 mm de Hg (30 Pa).

Par "huile non volatile", on entend une huile restant sur la peau à température ambiante et pression atmosphérique au moins plusieurs heures et ayant notamment une pression de vapeur inférieure ou égale à 0,01 mm de Hg (1,33 Pa).

Ces huiles volatiles ou non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'iso-hexadécane, et par exemple les huiles vendues sous les noms commerciaux d'Isopars^{®} ou de Permethyls^{®}, les esters ramifiés en C₈-C₁₆ tels que le néopentanoate d'iso-hexyle, et leurs mélanges. D'autres huiles hydrocarbonées volatiles comme les distillats de pétrole, notamment ceux vendus sous la dénomination Shell Soll^{®} par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans la composition selon l'invention à une teneur allant de 0,1 % à 90 % en poids, notamment de 1% à 50 % en poids, et en particulier de 2 % à 35 % en poids, par rapport au poids total de la composition.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer:
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/ caprique comme ceux vendus par la société Stéarineries Dubois ou ceux vendus sous les dénominations Miglyol 810^{®}, 812^{®} et 818^{®} par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane et leurs mélanges,
- les esters de synthèse comme les huiles de formule R₁ COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate' d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-buty-loctanol, et le 2-undécylpentadécanol,
- les acides gras supérieurs tels que l'acide oléique, l'acide linoléique, l'acide linolénique et leurs mélanges, et
- les carbonates de di-alkyle, les 2 chaînes alkyles pouvant être identiques ou différentes, tel que le dicaprylyl carbonate commercialisé sous la dénomination Cetiol CC^{®}, par Cognis.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates.

Les huiles non volatiles peuvent être présentes dans la composition selon l'invention en une teneur allant de 0,01 à 90 % en poids, notamment de 0,1 % à 85% en poids, et en particulier de 1 % à 70 % en poids, par rapport au poids total de la composition.

Outre une cire ou phase cireuse conforme à l'invention, la composition selon l'invention peut également comprendre une cire non conforme à l'invention.

La composition selon l'invention peut comprendre en outre au moins une cire distincte des cires conformes à l'invention. Elle peut être hydrocarbonée, fluorée et/ou siliconée et être d'origine animale, végétale, minérale ou synthétique. Elle peut être choisie par exemple parmi la cire d'abeille, la cire de Camauba, la cire de Candellila, les cires de paraffine, l'huile de ricin hydrogénée, les cires de silicone, et les cires microcristallines et leurs mélanges.

### Agent tensioactif

L'émulsion contient avantageusement au moins un agent tensioactif qui peut être présents notamment en une proportion allant de 0,1 à 30 % en poids, et mieux de 5% à 15% en poids, par rapport au poids total de la composition.

Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisables dans la composition selon l'invention peuvent être choisis parmi :
- les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆alkyl glucose polyoxyéthylénés, et leurs mélanges,
- lestensioactifs anioniques : les acides gras en C₁₆-C₃₀ neutralisés parles amines, l'ammoniaque ou les sels alcalins, et leurs mélanges.

Conviennent plus particulièrement des tensioactifs permettant l'obtention d'émulsion eau-dans-huile.

Les tensioactifs permettant l'obtention d'émulsions eau-dans-huile sont les tensioactifs donc la HLB (balance hydrophile / lipophile) est comprise entre 3 et 6. La définition de la HLB figure dans le livre Galenica 5, Les Systèmes Dispersés-I Agents de Surface et Emulsions, F. Puisieux, M. Seiller, Pages 153-155, Editions Lavoisier.

Plus spécifiquement, des tensioactifs siliconés de type diméthicone copolyol ou alkyl diméthicone copolyol peuvent être utilisés, comme ceux commercialisés sous les dénominations Abil EM90^{®}, Abil WE09^{®} (de la société Goldschmidt) et DC3225C^{®}, DC5200^{®} (par la société Dow Coming).

### Phase particulaire

La composition de l'invention, peut en outre comprendre une phase particulaire pouvant être présente à raison de 0,01 % à 40 % en poids, notamment de 0,01 % à 30 % en poids et en particulier de 0,05 % à 20 % en poids, par rapport au poids total de la composition.

Elle peut notamment comprendre des pigments et/ou des nacres et/ou des charges classiquement utilisés dans les compositions cosmétiques.

Par « pigments », il faut comprendre des particules blanches ou colorées, minérales ou organiques, insolubles dans la phase hydrophile liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées.

Les pigments peuvent être présents dans la composition à raison de 0,01 % à 25 % en poids, en particulier de 0,01 % à 5 % en poids, et notamment de 0,02 % à 5 % en poids par rapport au poids de la composition.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome. Parmi les pigments organiques utilisables dans l'invention, on peut citer le noir de carbone, les pigments de type D & C, et les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Les nacres peuvent être présentes dans la composition à raison de 0,01 % à 25 % en poids, notamment de 0,01 % à 15 % en poids, et en particulier de 0,02 % à 5 % en poids, par rapport au poids total de la composition.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être présentes à raison de 0, 01 à 40 % en poids, notamment 0,01 à 30 % en poids, et en particulier de 0,02 % à 20 % en poids par rapport au poids total de la composition.

Il peut notamment s'agir de charges sphériques comme par exemple le talc, le stéarate de zinc, le mica, le kaolin, les poudres de polyamide (Nylon^{®}) (Orgasol^{®} de chez Atochem), les poudres de polyéthylène, les poudres de polymères de tétrafluoroéthylène (Téflon^{®}), l'amidon, le nitrure de bore, des microsphères polymériques telles que celles de chlorure de polyvinylidène/acrylonitrile comme l'Expancel^{®} (Nobel Industrie), de copolymères d'acide acrylique (Polytrap^{®} de la société Dow Corning), les microbilles de résine de silicone (Tospearls^{®} de Toshiba, par exemple), et les organopolysiloxanes élastomères.

La composition peut comprendre également des colorants hydrosolubles ou liposolubles en une teneur allant de 0,01 % à 6 % en poids, par rapport au poids total de la composition, notamment allant de 0,01 % à 3 % en poids. Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, et le jaune quinoléine. Les colorants hydrosolubles sont par exemple le jus de betterave et le bleu de méthylène.

La composition selon l'invention peut, de plus, comprendre tous les ingrédients classiquement utilisés dans les domaines concernés et plus spécialement dans le domaine cosmétique et dermatologique. Ces ingrédients sont en particulier choisis parmi les vitamines, les antioxydants, les oligo-éléments, les adoucissants, les séquestrants, les parfums, les agents alcalinisants ou acidifiants, les conservateurs, les filtres UV, les actifs hydrophiles ou lipophiles et leurs mélanges. Les quantités de ces différents ingrédients sont celles classiquement utilisées dans les domaines concernés et par exemple de 0,01 % à 20 % du poids total de la composition.

Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction considérée.

La composition de l'invention peut être obtenue selon les procédés de préparation classiquement utilisés en cosmétique ou en dermatologie. Plus précisément les émulsions conformes à l'invention sont préparées sous des protocoles de préparation d'émulsion eau-dans-huile conventionnels.

Compte tenu de la présence dans ces émulsions de cire, notamment d'au moins une cire possédant un point de fusion variant de 25 à 40°C, l'émulsification est généralement conduite à une température supérieure d'au moins 5°C à la température de fin de fusion de la cire la plus élevée.

Plus précisément, l'ensemble des ingrédients lipidiques et/ou liposolubles sont mélangés et portés à une température supérieure d'au moins 5 °C à la température de fin de fusion de la cire ou phase cireuse. La phase aqueuse, associée aux composants hydrosolubles, est également portée à une température équivalente. On procède ensuite à l'incorporation progressive, généralement goutte à goutte, de la phase aqueuse dans la phase grasse et l'ensemble est homogénéisé sous agitation, avant d'être laissé refroidir à température ambiante.

Les compositions selon l'invention peuvent se représenter sous la forme d'un produit coulé en stick ou en coupelle comme par exemple les rouges à lèvres ou les baumes à lèvres, les fonds de teintcoulés, les produits anticernes, les correcteurs et/ou embellisseurs de teints et fards à paupières ou à joue, les baumes antisolaires et les baumes déodorants.

Les exemples de compositions ci-après sont donnés à titre illustratif et sont à caractère limitatif. Sans précision contraire, les pourcentages sont exprimés en pourcentages pondéraux et les plages de valeurs exprimées sous la forme « entre ... et ... » incluent les valeurs figurant les bornes précisées.

### EXEMPLES

Sept formulations de fonds de teint en stick sont préparées selon le protocole suivant.

Dans un poêlon sont introduits les composants de la phase grasse, à savoir les cires, huiles, tensioactifs et pigments. L'ensemble est porté à une température suffisante pour fondre l'intégralité des cires. Cette température au moins supérieure de 5 °C à celle du point de fusion de la cire le plus élevé. Les composants de la phase aqueuse, à savoir l'eau, les conservateurs et sels sont mélangés et chauffés (à une température équivalente). On procède ensuite à l'ajout de goutte à goutte de la phase aqueuse, à la phase grasse, environ 20 g d'eau par minute, sous agitation avec un appareillage Rayneri à 400 rpm.

A la fin de l'ajout, l'agitation est maintenue vingt minutes à 1000 rpm. L'émulsion est ensuite coulée dans des conditionnements pour sticks de marque Laffon et de diamètre extérieur 25 mm.

La cire utilisée à l'état de cristallites conformes à l'invention est la cire commercialisée sous la dénomination BELSIL DMC 6038 par la société Wacker. Elle est mise en oeuvre dans les essais B, D et G ci-après.

Les autres essais sont des essais comparatifs c'est-à-dire n'incorporant pas de cristallites de cire de facteur de forme au moins égale à 2 dans leur émulsion.

| | | A comparatif | B | C comparatif | D | E comparatif | F comparatif | G |
|---|---|---|---|---|---|---|---|---|
| phase cireuse | Huile de jojoba hydrogénée (société Desert Whale) | 20 | 16 | | | 9 | | |
| | Cire de polyéthylène (PERFORMALENE 400 POLYETHYLENE de New Phase Technology) | | | 20 | 16 | | 9 | 7 |
| | BIS-PEG-15 METHYL ETHER DIMETHICONE (BELSIL DMC 6038 de la société Wacker) | | 4 | | 4 | | | 2 |
| phase huileuse | Polydiméthylsiloxane (FLUID DC 200 10 CST de Dow Corning) | 19 | 19 | | | 5 | | |
| | Isoparaffine hydrogénée (Parléam de la société NOF Corporation) | | | 19 | 19 | | 5 | 5 |
| tensioactif | Tensioactif siliconé (Abil WE09 de la société Goldschmidt) | 5 | 5 | 5 | 5 | 5 | 5 | 5 |
| pigments | Oxyde de fer jaune | 5 | 5 | 5 | 5 | 5 | 5 | 5 |
| phase aqueuse | | | | | | | | |
| eau | | 25 | 25 | 25 | 25 | 50 | 50 | 50 |
| glycérol | | 25 | 25 | 25 | 25 | 25 | 25 | 25 |
| conservateur | Para hydroxy benzoate de méthyle | 0,3 | 0,3 | 0,3 | 0,3 | 0,3 | 0,3 | 0,3 |
| sel | | 0,7 | 0,7 | 0,7 | 0,7 | 0,7 | 0,7 | 0,7 |
| dureté mesurée | | 0,904 | 0,317 | 0,938 | 0,926 | 0,269 | 0,713 | 0,426 |
| Formulation sous forme de stick | | possible | possible | possible | possible | trop mou, impossible | possible | possible |
| Aspect dépôt | | faible | important | faible | important | - | très faible | important |

La qualité du dépôt est appréciée en comparatif après application en un seul passage sur l'avant-bras, un dépôt suffisamment important est recherché car il permet d'apporter une couleur visible sur la peau.

Seuls les 3 essais selon l'invention à savoir B, D et G permettent d'obtenir simultanément l'obtention d'un stick de texture convenable et un dépôt satisfaisant avec dans le cas de l'essai G, une quantité importante en phase aqueuse.

## Revendications

1. Composition cosmétique sous la forme d'une émulsions solide eau-dans-hule comprenant une phase aqueuse dispersée dans une phase grasse **caractérisée en ce qu'**elle comprend plus de 60 % en poids de phase aqueuse et **en ce que** ladite phase grasse comprend au moins une cire dont la température de fusion est comprise entre 25 ° et 42 °C, et qui se trouve à l'état solide sous la forme de cristrallites possédant un facteur de forme au moins égal à 2 et une longueur moyenne comprise entre 20 µm et 50 µm.

2. Composition cosmétique selon la revendication 1, **caractérisée en ce que** les cristallites possèdent un facteur de forme supérieur ou égal à 3, notamment supérieur ou égal à 4 et plus particulièrement supérieur ou égal à 5.

3. Composition cosmétique selon l'une quelconque des revendications 1 à 2, caractérisée ce que ladite cire possède une température de fusion comprise entre 25 et 40 °C.

4. Composition selon l'une quelconque des revendications 1 à 3, **caractérisée en ce que** la cire possède une température de fusion comprise entre 25 et 35 °C.

5. Composition selon l'une quelconque des revendications précédentes, **caractérisée en ce que** les cristallites possèdent une longueur moyenne comprise entre 30 µm et 50 µm.

6. Composition selon l'une quelconque des revendications précédentes, **caractérisée en ce qu'**elle comprend plus de 70 % en poids, ou encore plus de 75 % en poids, voire plus de 30 % en poids de phase aqueuse.

7. Composition selon l'une quelconque des revendications 1 à 6 **caractérisée en ce que** la phase grasse comprend de 10 à 40 % en poids, notamment de 10 à 30 % en poids, en particulier de 5 à 25 % en poids d'une phase cireuse contenant de 50 à 100 %, en particulier de 70 à 100 % en poids de ladite cire.

8. Composition selon l'une quelconque des revendications précédentes, **caractérisée en ce qu'**elle comprend de 1 à 20 % en poids notamment de 2 à 20 % en poids, en particulier de 4 à 10 % en poids de ladite cire.

9. Composition selon l'une quelconque des revendications précédentes, **caractérisée en ce que** ladite cire est au moins une cire de polyméthylène.

10. Composition selon l'une quelconque des revendications précédentes, **caractérisée en ce que** ladite cire est au moins une silicone polyoxyalkylénée.

11. Composition selon la revendication 10, caractérisée en ce q'il s'agit d'au moins une silicone polyoxyalkylénée de formule III 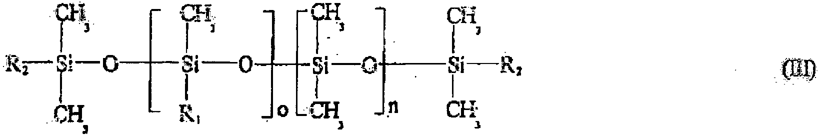 dans laquelle :
- R₁, identique ou différent, représente un radical alkyle, linéaire ou ramifié en C₁-C₃₀ ou phényle,
- R₂, identique ou différent, représente un radical C_{c}H_{2c}-O-(C₂H₄O)₃(C₃H₅O)_{b}-R_{5,}
- R₅, identique ou différent, est choisi parmi un atome d'hydrogène, un radical alkyle, linéaire ou ramifié, de 1 à 12 atomes de carbone, un radical alcoxy, linéaire ou ramifié; de 1 à 6 atomes de carbone, un radical acyle, linéaire ou ramifié, de 2 à 30 atomes de carbone, un radical hydroxyle, aminoalcoxy en C₁-C₆ éventuellement substitué sur l'amine, aminoacyle en C₂-C₆ éventuellement substitué sur l'amine, aminoalkyle éventuellement substitué sur l'amine et sur la chaîne alkyle, carboxyacyle en C₂-C₃₀, un groupement éventuellement substitué par un ou deux radicaux aminoalkyle substitués, -NHCO(CH₂)_{d}OH, un groupement phosphate,
- d varie de 1 à 10,
- n varie de 0 à 500,
- o varie de 0 à 20,
- a varie de 0 à 50,
- b varie de 0 à 50,
- a + b est supérieur ou égal à 2,
- c varie de 0 à 4.

12. Composition selon l'une quelconque des revendications précédentes, **caractérisée en ce qu'**elle comprend de 0,5 à 80 % en poids, en particulier de 1 à 75 % en poids, plus particulièrement de 2 à 65 % en poids, voire de 3 à 60 % en poids d'au moins une phase grasse liquide par rapport au poids total de la composition.

13. Composition selon l'une quelconque des revendications précédentes, **caractérisée en ce qu'**elle se présente sous la forme d'un produit coulé en stick ou en coupelle.

14. Composition selon l'une quelconque des revendications précédentes, **caractérisée en ce qu'**elle se présente sous la forme de rouges à lèvres, baumes à lèvres, fonds de teint coulés, produits anti-cernes, produits « correcteurs » ou « embellisseurs » de teint, et/ou fards à paupières ou à joues, baumes antisolaires ou baumes déodorants.

15. Utilisation d'au moins une cire possédant une température de fusion comprise entre 25 et 42 °C et se présentant à l'état de solide sous la forme de cristallites possédant un facteur de forme au moins égal à 2 et une longueur moyenne comprise entre 20 µm et 50 µm, à titre d'agent texturant pour la préparation d'une composition cosmétique sous la forme d'une émulsion solide eau-dans-huile comprenant plus de 60 % en poids de phase aqueuse.

16. Procédé cosmétique de soin et/ou de maquillage de la peau et/ou des lèvres comprenant l'application sur la peau et/ou les lèvres d'une composition telle que définie selon l'une quelconque des revendications 1 à 14.

## Patentansprüche

1. Kosmetische Zusammensetzung in Form einer festen Wasser-in-ÖI Emulsion, die eine wässrige Phase, dispergiert in einer fetten Phase, beinhaltet, **dadurch gekennzeichnet, dass** sie mehr als 60 Gew.-% wässrige Phase beinhaltet und dadurch dass die fette Phase mindestens ein Wachs beinhaltet, dessen Schmelztemperatur zwischen 25 und 42 °C beträgt und die im festen Zustand in der Form von Kristalliten vorliegt, die einen Formfaktor von mindestens gleich 2 und eine durchschnittliche Länge zwischen 20 µm und 50 µm besitzen.

2. Kosmetische Zusammensetzung nach Anspruch 1, **dadurch gekennzeichnet, dass** die Kristalliten einen Formfaktor von größer oder gleich 3, insbesondere größer oder gleich 4 und ganz besonders größer oder gleich 5 besitzen.

3. Kosmetische Zusammensetzung nach einem der Ansprüche 1 bis 2, **dadurch gekennzeichnet, dass** das Wachs eine Schmelztemperatur zwischen 25 und 40 °C besitzt.

4. Zusammensetzung nach einem der Ansprüche 1 bis 3, **dadurch gekennzeichnet, dass** das Wachs eine Schmelztemperatur zwischen 25 und 35 °C besitzt.

5. Zusammensetzung nach einem der vorangehenden Ansprüche, **dadurch gekennzeichnet, dass** die Kristallite eine durchschnittliche Länge zwischen 30 µm und 50 µm besitzen.

6. Zusammensetzung nach einem der vorangehenden Ansprüche, **dadurch gekennzeichnet, dass** sie mehr als 70 Gew.-% oder auch mehr als 75 Gew.-%, sogar mehr als 80 Gew.-% wässrige Phase beinhaltet.

7. Zusammensetzung nach einem der Ansprüche 1 bis 6, **dadurch gekennzeichnet, dass** die fette Phase von 10 bis 40 Gew.-%, insbesondere von 10 bis 30 Gew.-%, speziell von 5 bis 25 Gew.-% einer wachsartigen Phase beinhaltet, die von 50 bis 100 % insbesondere von 70 bis 100 Gew.-% des Wachses enthält.

8. Zusammensetzung nach einem der vorangehenden Ansprüche, **dadurch gekennzeichnet, dass** sie von 1 bis 20 Gew.-%, insbesondere von 2 bis 20 Gew.-%, speziell von 4 bis 10 Gew.-% des Wachses enthält.

9. Zusammensetzung nach einem der vorangehenden Ansprüche, **dadurch gekennzeichnet, dass** das Wachs mindestens ein Polymethylenwachs ist.

10. Zusammensetzung nach einem der vorangehenden Ansprüche, **dadurch gekennzeichnet, dass** das Wachs mindestens ein Polyoxyalkylensilikon ist.

11. Zusammensetzung nach Anspruch 10, **dadurch gekennzeichnet, dass** es sich um mindestens ein Polyoxyalkylensilikon der Formel III handelt 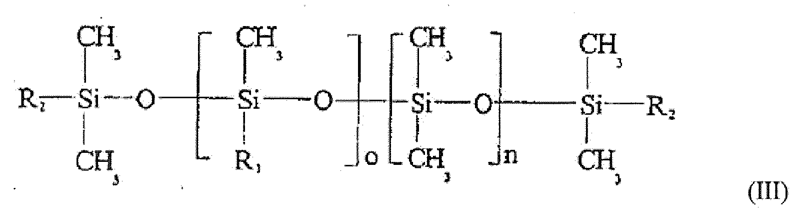 wobei:
- R₁ gleich oder verschieden ist und einen linearen oder verzweigten C₁-C₃₀-Alkyl- oder Phenylrest darstellt,
- R₂ gleich oder verschieden ist und einen C_{c}H_{2c}-O-(C₂H₄O)ₐ(C₃H₆O)_{b}-R₅-Rest darstellt,
- R₅ gleich oder verschieden ist und ausgewählt ist aus einem Wasserstoffatom, einem linearen oder verzweigten Alkylrest mit 1 bis 12 Kohlenstoffatomen, einem linearen oder verzweigten Alkoxy-Rest mit 1 bis 6 Kohlenstoffatomen, einem linearen oder verzweigten Acyl-Rest mit 2 bis 30 Kohlenstoffatomen, einem Hydroxyl-, einem C₁-C₆-Aminoalkoxy-Rest, der gegebenenfalls am Amin substituiert ist, einem C₂-C₆-Aminoacyl-Rest, der gegebenenfalls am Amin substituiert ist und einem Aminoalkylrest, der gegebenenfalls. am Amin und an der Alkylkette substituiert ist, einem C₂-C₃₀-Carboxyacylrest, einer Gruppe, die gegebenenfalls mit einem oder zwei substituierten Aminoalkyl-Resten substituiert ist, -NHCO(CH₂)_{d}OH, einer Phosphatgruppe,
- d von 1 bis 10 variiert,
- n von 0 bis 500 variiert,
- o von 0 bis 20 variiert,
- a von 0 bis 50 variiert,
- b von 0 bis 50 variiert,
- a + b größer oder gleich 2 ist, und
- c von 0 bis 4 variiert.

12. Zusammensetzung nach einem der vorstehenden Ansprüche, **dadurch gekennzeichnet, dass** sie von 0,5 bis 80 Gew.-%, insbesondere von 1 bis 75 Gew.% und noch spezieller von 2 bis 65 Gew.-%, sogar von 3 bis 60 Gew.-% mindestens einer fetten, flüssigen Phase bezogen auf das Gesamtgewicht der Zusammensetzung beinhaltet.

13. Zusammensetzung nach einem der vorstehenden Ansprüche, **dadurch gekennzeichnet, dass** sie in Form eines zu einem Stift oder in Schälchen gegossenen Produkts vorliegt.

14. Zusammensetzung nach einem der vorstehenden Ansprüche, **dadurch gekennzeichnet, dass** sie in Form von Lippenstiften, Lippenbalsam, Korzapakt-Make-up, Präparaten gegen Augenringe, Präparaten zur "Korrektur" oder "Verschönerung" des Teints, und/oder Lidschatten oder Wangenrot, Präparaten gegen Sonne oder Deodorants vorliegt.

15. Verwendung von mindestens einem Wachs, das eine Schmelztemperatur zwischen 25 und 42 °C aufweist und in festem Zustand in Form von Kristalliten mit einem Formfaktor von mindestens gleich 2 und einer durchschnittlichen Länge von 20 µm bis 50 µm als strukturgebendes Mittel vorliegt, für die Herstellung einer kosmetischen Zusammensetzung in Form einer festen Wasser-in-Öl Emulsion, die mehr als 60 Gew.-% wässrige Phase beinhaltet.

16. Kosmetisches Verfahren zur Pflege und/oder zum Schminken der Haut und/oder der Lippen, das die Anwendung einer Zusammensetzung wie in einem der Ansprüche 1 bis 14 definiert, auf der Haut und/oder den Lippen beinhaltet.

## Claims

1. A cosmetic composition in the form of a solid water-in-oil emulsion comprising an aqueous phase dispersed in a fatty phase, **characterized in that** it comprises more than 60 weight % of aqueous phase and **in that** said fatty phase comprises at least one wax having a melting point between 25° and 42 °C and is in the solid state in the form of crystallites having a form factor of at least 2 and mean length of between 20 µm and 50 µm.

2. The cosmetic composition according to claim 1, **characterized in that** the crystallites have a form factor of 3 or higher, in particular 4 or higher and more particularly 5 or higher.

3. The cosmetic composition according to any of claims 1 to 2, **characterized in that** said wax has a melting point of between 25 and 40 °C.

4. The composition according to any of claims 1 to 3, **characterized in that** the wax has a melting point between 25 and 35 °C.

5. The composition according to any of the preceding claims, **characterized in that** the crystallites have a mean length of between 30 µm and 50 µm.

6. The composition according to any of the preceding claims, **characterized in that** it comprises more than 70 weight %, or more than 75 weight %, even more than 80 weight % of aqueous phase.

7. The composition according to any of claims 1 to 6, **characterized in that** the fatty phase comprises 10 to 40 weight %, in particular 10 to 30 weight %, more particularly 5 to 25 weight % of wax phase containing 50 to 100 weight %, in particular 70 to 100 weight % of said wax.

8. The composition according to any of the preceding claims, **characterized in that** it comprises 1 to 20 weight %, in particular 2 to 20 weight %, more particularly 4 to 10 weight % of said wax.

9. The composition according to any of the preceding claims, **characterized in that** said wax is at least a polymethylene wax.

10. The composition according to any of the preceding claims, **characterized in that** said wax is at least a polyoxyalkylene silicone.

11. The composition according to claim 10, **characterized in that** it is at least a polyoxyalkylene silicone of formula III: 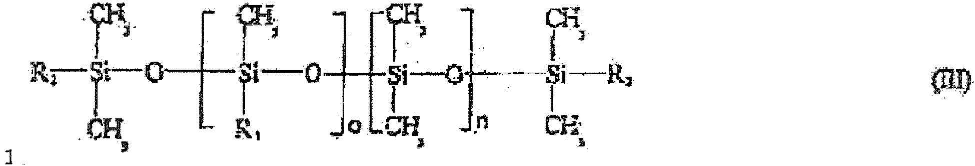 wherein:
- R₁, the same or different, is a straight-chain or branched C₁-C₁₀ alkyl radical or phenyl radical;
- R₂, the same or different, is a C_{c}H_{2c}-O-(C₂H₄O)ₐ(C₃H₆O)_{b}-R₅ radical;
- R₅, the same or different, is selected from among a hydrogen atom, a straight-chain or branched alkyl radical having 1 to 12 carbon atoms, a straight-chain or branched alkoxy radical having 1 to 6 carbon atoms, a straight-chain or branched acyl radical having 2 to 30 carbon atoms, a hydroxyl radical, C₁-C₆ aminoalkoxy radical optionally substituted on the amine, C₂-C₆ aminoacyl radical optionally substituted on the amine, aminoalkyl radical optionally substituted on the amine and on the alkyl chain, C₂-C₃₀ carboxyacyl radical, a group optionally substituted by one or two substituted aminoalkyl radicals, -NHCO(CH₂)_{d}OH, a phosphate group,
- d varies from 1 to 10,
- n varies from 0 to 500
- o varies from 0 to 20
- a varies from 0 to 50
- b varies from 0 to 50
- a + b equals 2 or higher
- c varies from 0 to 4.

12. The composition according to any of the preceding claims, **characterized in that** it comprises 0.5 to 80 weight %, in particular 1 to 75 weight %, more particularly 2 to 65 weight %, even 3 to 60 weight % of at least one liquid fatty phase relative to the total weight of the composition.

13. The composition according to any of the preceding claims, **characterized in that** it is in the form of a product cast in stick or dish form.

14. The composition according to any of the preceding claims, **characterized in that** it is in the form of a dipstick, lip balm, cake foundation, under-eye concealer, skin corrector or enhancer and/or eye shadow or blusher, sun protection balm or deodorant balm.

15. The use of at least one wax having a melting point between 25 and 42°C and in the solid state in the form of crystallites having a form factor of at least 2 and mean length of between 20 µm and 50 µm as texturizing agent to prepare a cosmetic composition in the form of a solid water-in-oil emulsion comprising more than 60 weight % of aqueous phase.

16. A cosmetic care and/or make-up method for the skin and/or lips comprising the application to the skin and/or lips of a composition such as defined in any of claims 1 to 14.
